# EUROPEAN PATENT APPLICATION

(11) **EP 2 924 024 A2**
(43) Date of publication of application: **30.09.2015**
(21) Application number: 15160102.8
(22) Date of filing: 20.03.2015
(51) Int. Cl.: C07D 223/16, A61K 31/55, A61P 3/04, A61P 25/30, A61P 25/24

(54) **SOLID FORMS OF LORCASERIN HYDROCHLORIDE**

(30) Priority: 21.03.2014 US 201461968674 P; 04.04.2014 US 201461975397 P; 16.05.2014 US 201461994497 P
(71) Applicant: Medichem, S.A., 08970 Barcelona (ES)
(72) Inventor: Duran López, Ernesto, 08755 Castellbisbal (ES); Benito Vélez, Mónica, 08902 L'Hospitalet de Llobregat (ES)
(74) Representative: ABG Patentes, S.L.

(57) **Abstract**

The invention relates to lorcaserin hydrochloride, processes of preparing lorcaserin hydrochloride, mixtures comprising lorcaserin hydrochloride and at least one pharmaceutical excipient and pharmaceutical compositions and therapeutic uses thereof. The invention also relates to cocrystals comprising lorcaserin, processes of preparing thereof, and pharmaceutical compositions and therapeutic uses thereof.

## Description

### FIELD OF THE INVENTION

This invention relates to lorcaserin hydrochloride, processes of preparing lorcaserin hydrochloride, mixtures comprising lorcaserin hydrochloride and at least one pharmaceutical excipient and pharmaceutical compositions and therapeutic uses thereof. In particular, the present invention relates to lorcaserin hydrochloride in amorphous form.

This invention also relates to cocrystals comprising lorcaserin, processes of preparing thereof, and pharmaceutical compositions and therapeutic uses thereof. In particular, the present invention relates to a cocrystal comprising lorcaserin and urea and a cocrystal compising lorcaserin and mandelic acid.

### BACKGROUND OF THE INVENTION

Lorcaserin (compound I) is the international commonly accepted non-proprietary name (INN) for (*R*)-8-chloro-1-methyl-2,3,4,5-tetrahydro-1*H*-3-benzazepine, and has an empirical formula of CₙH₁₄NCl and a molecular weight of 195.69.

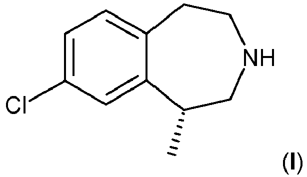

The hydrochloride salt of lorcaserin is known to be therapeutically useful and is commercially marketed as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (e.g., hypertension, dyslipidemia, type 2 diabetes). In the United States, lorcaserin hydrochloride (as hemihydrate) is marketed under the name Belviq^{™}.

The hydrochloride salt of lorcaserin was first described in U.S. Patent No. 8,367,657. Concretely, Example 14 of this patent discloses the stirring of the free base of lorcaserin with hydrochloric acid in a mixture of dichloromethane and ether, at room temperature, followed by evaporation of the solvent under reduced pressure to give a white solid which corresponds to the hydrochloric acid salt of lorcaserin.

U.S. Patent No. 8,168,624 is related to crystalline forms of the hydrochloride salt of lorcaserin. Example 1 of this patent is identical to Example 14 of U.S. Patent No. 8,367,657. Additionally, the patent discloses two anhydrous forms of lorcaserin hydrochloride, *i.e.* Form I and Form II, and a hemihydrate form (Form III). The patent also discloses that anhydrous forms I and II are hygroscopic forms, both of which readily convert to the hemihydrate (Form III) upon exposure to moisture. Moreover, Example 6 of this patent discloses that the hemihydrate (Form III) partially converts into anhydrous Form I after heating at 60 °C for one day, or at 80 °C for 30 minutes.

Patent application WO 2011/153206 A1 discloses additional crystalline forms of the hydrochloride salt of lorcaserin. Based on their X-ray powder diffraction (XRPD) patterns and differential scanning calorimetry (DSC) plots, Form II as disclosed in patent application WO 2011/153206 A1 is equivalent to Form I of U.S. Patent No. 8,168,624, and Form III (hemihydrate) as disclosed in patent application WO 2011/153206 A1 is also equivalent to Form III of U.S. Patent No. 8,168,624. The dynamic moisture sorption (DMS) plot of anhydrous forms I and IV as disclosed in figures 3 and 14, respectively, of patent application WO 2011/153206 A1 are substantially similar to the DMS plot of Form II as disclosed in Figure 7 of the same patent application, thus indicating substantial adsorption of moisture at relative humidity values above about 50% for all three anhydrous forms. Consequently, both anhydrous forms I and IV as disclosed in patent application WO 2011/153206 A1 are hygroscopic forms which readily convert to the hemihydrate (Form III) upon exposure to moisture, similarly to anhydrous Form II as disclosed in patent application WO 2011/153206 A1 which is equivalent to Form I of U.S. Patent No. 8,168,624. Additionally, Example 6 of patent application WO 2011/153206 A1 discloses that anhydrous forms I and II are monotropically related and, consequently, transformation of one of these forms into the other one is expected when heating above or cooling below their corresponding transition temperature, which is between 44 and 50 °C based on Figure 17 of the same patent application. Similarly, anhydrous forms II and IV are also monotropically related and, consequently, transformation of one of these forms into the other one is expected when heating above or cooling below their corresponding transition temperature, which is between 80 and 90 °C based on Figure 17 of the same patent application.

Example 1 of patent application WO 2012/030927 A2 discloses a modified-release tablet comprising Form III (hemihydrate) of lorcaserin hydrochloride.

In view of the foregoing, there is an unmet need for forms of lorcaserin hydrochloride having an enhanced physical stability, and for pharmaceutical compositions comprising these forms of lorcaserin hydrochloride having an enhanced physical stability.

### BRIEF DESCRIPTION OF THE DRAWINGS

**Figure 1** depicts the X-Ray Powder Diffraction (XRPD) plot of amorphous lorcaserin hydrochloride as obtained in Example 4.
**Figure 2** depicts the X-Ray Powder Diffraction (XRPD) plot of amorphous lorcaserin hydrochloride as obtained in Example 6.
**Figure 3** depicts the X-Ray Powder Diffraction (XRPD) plot of the cocrystal of lorcaserin hydrochloride and urea.
**Figure 4** depicts the X-Ray Powder Diffraction (XRPD) plot of the cocrystal of lorcaserin hydrochloride and L-(+)-mandelic acid.

### BRIEF SUMMARY OF THE INVENTION

The present invention relates to lorcaserin hydrochloride in amorphous form, to mixtures comprising thereof, to pharmaceutical compositions comprising thereof, to processes for the preparation thereof, and to the use thereof for preparing a medicament for chronic disease treatment.

In particular, the invention relates to lorcaserin hydrochloride in amorphous form, to mixtures comprising lorcaserin hydrochloride in amorphous form, to pharmaceutical compositions comprising lorcaserin hydrochloride in amorphous form, and to processes for the preparation of mixtures comprising lorcaserin hydrochloride in amorphous form.

This invention also relates to cocrystals comprising lorcaserin, processes of preparing thereof, and pharmaceutical compositions and therapeutic uses thereof.

In particular, the present invention relates to a cocrystal comprising lorcaserin and urea and a cocrystal compising lorcaserin and mandelic acid.

### DETAILED DESCRIPTION OF THE INVENTION

There is now provided by the present invention lorcaserin hydrochloride with improved properties.

In one aspect, the lorcaserin hydrochloride of the present invention can be characterized and distinguished from the prior art by reference to its physical form. Particularly, the lorcaserin hydrochloride of the present invention is in amorphous form.

An amorphous material is a non-crystalline solid that lacks the long-range order characteristic of a crystal. While a crystal is composed of periodically arranged atoms in a three dimensional space, amorphous materials do not possess that periodicity and atoms are randomly distributed. This lack of three-dimensional periodicity is reflected in the materials' inability to diffract X-rays constructively; as such analysis of an amorphous sample by X-ray powder diffraction (XRPD) gives rise to a diffuse profile devoid of the characteristic peaks observed for crystalline phases. Since X-rays become scattered in many directions, a large bump distributed in a wide range of angles (2-theta) is obtained as a result of the XRPD analysis.

In a preferred embodiment, the amorphous lorcaserin hydrochloride of the present invention is characterized by having an X-ray powder diffraction (XRPD) pattern showing no detectable peaks in the range from 2 to 50° (2θ or 2-theta).

As used in the present invention, the term "detectable peak" denotes that the peak in the diffraction plot has a signal-to-noise (S/N) ratio equal or higher than 3.0. Signal-to-noise ratio of a peak is a dimensionless parameter that is calculated by dividing the height of the peak by the baseline width of the diffraction plot, both expressed using the same length units (*e.g.* mm). The height of a peak is calculated by measuring the distance between peak's maximum and the baseline of the peak. Peak's maximum 2-theta values are identified by having a first-derivative value equal to zero, and a negative second-derivative value. The baseline of the peak is obtained by tracing a straight line which is tangent to the diffraction plot at the closest 2-theta value which is lower than peak's maximum 2-theta value and has both first- and second-derivative values equal to zero, and also tangent to the diffraction plot at the closest 2-theta value which is higher than peak's maximum 2-theta value and has both first- and second-derivative values equal to zero. The height of the peak is obtained by tracing a second straight line which is parallel to the previously obtained baseline of the peak and tangent to the diffraction plot at the peak's maximum 2-theta value, and measuring the distance (perpendicularly to the X-axis of the diffraction plot) between both parallel lines. On the other hand, the baseline width of the diffraction plot is calculated by tracing two parallel lines to the X-axis of the diffraction plot, the first line being tangent to the diffraction plot at its maximum value in the range between 45 and 50° (2-theta), and the second line being tangent to the diffraction plot at its minimum value in the same range between 45 and 50° (2-theta), and measuring the perpendicular distance between both parallel lines.

In a preferred embodiment, the amorphous lorcaserin hydrochloride of the present invention is characterized by having an X-ray powder diffraction (XRPD) pattern showing no peaks with a signal-to-noise (S/N) ratio equal or higher than 3.0 in the range from 2 to 50° (2-theta).

Crystalline forms of lorcaserin hydrochloride are characterized in the prior art with respect to their characteristic peaks in the X-ray powder diffraction (XRPD) pattern. Precisely, U.S. Patent No. 8,168,624 describes that crystalline Form I of lorcaserin hydrochloride is characterized by having characteristic peaks at 6.5, 9.6 and 10.2° ± 0.2° (2-theta), crystalline Form II of lorcaserin hydrochloride is characterized by having a characteristic peak at 11.4° ± 0.2° (2-theta), and crystalline Form III of lorcaserin hydrochloride is characterized by having characteristic peaks at 13.7° and 14.9° ± 0.2° (2-theta). Additionally, patent application WO 2011/153206 A1 describes that crystalline Form I of lorcaserin hydrochloride is characterized by having characteristic peaks at 11.5, 22.4, 22.9, 23.0, 23.3, 23.6, 23.9, 25.5, 27.6, 27.7, 28.0 and 29.8° ± 0.2° (2-theta), crystalline Form II of lorcaserin hydrochloride is characterized by having characteristic peaks at 12.9, 19.5, 19.8, 21.3, 21.6, 24.8 and 25.9° ± 0.2° (2-theta), and crystalline Form IV of lorcaserin hydrochloride is characterized by having characteristic peaks at 15.3 and 20.2° ± 0.2° (2-theta). A selection of the most characteristic peaks of the different crystalline forms of lorcaserin hydrochloride can also be obtained from their corresponding XRPD patterns, as disclosed in U.S. Patent No. 8,168,624 and WO 2011/153206 A1. Table 1 below summarizes the most characteristic peaks of the different crystalline forms of lorcaserin hydrochloride as disclosed in U.S. Patent No. 8,168,624 and WO 2011/153206 A1.

**Table 1**

| **Crystalline form** | **Most characteristic peaks (2-theta ± 0.2°)** |
|---|---|
| Form I of U.S. 8,168,624 (equivalent to Form II of WO 2011/153206 A1) | 12.9, 21.6 |
| Form II of U.S. 8,168,624 | 11.4 |
| Form III of U.S. 8,168,624 and WO 2011/153206 A1 | 13.7, 14.9 |
| Form I of WO 2011/153206 A1 | 11.5 |
| Form IV of WO 2011/153206 A1 | 15.3, 20.2 |

In a preferred embodiment, the amorphous lorcaserin hydrochloride of the present invention is characterized by having an X-ray powder diffraction (XRPD) pattern showing no detectable peaks corresponding to the crystalline forms of lorcaserin hydrochloride.

In a further preferred embodiment, the amorphous lorcaserin hydrochloride of the present invention is characterized by having an X-ray powder diffraction (XRPD) pattern showing no detectable peaks at 11.4, 11.5, 12.9, 13.7, 14.9, 15.3, 20.2 or 21.6° ± 0.2° (2-theta). Precisely, the amorphous lorcaserin hydrochloride of the present invention is characterized by having an X-ray powder diffraction (XRPD) pattern showing no peaks with a signal-to-noise (S/N) ratio equal or higher than 3.0 at 11.4, 11.5, 12.9, 13.7, 14.9, 15.3, 20.2 or 21.6° ± 0.2° (2-theta).

The present invention further provides a mixture comprising the amorphous lorcaserin hydrochloride as hereinbefore described, together with at least one pharmaceutical excipient.

In a preferred embodiment, the mixture of the present invention is characterized by having an X-ray powder diffraction (XRPD) pattern showing no detectable peaks in the range from 2 to 50° (2θ or 2-theta). Precisely, the mixture of the present invention is characterized by having an X-ray powder diffraction (XRPD) pattern showing no peaks with a signal-to-noise (S/N) ratio equal or higher than 3.0 in the range from 2 to 50° (2-theta).

In a further preferred embodiment, the mixture of the present invention is characterized by having an X-ray powder diffraction (XRPD) pattern showing no detectable peaks at 11.4, 11.5, 12.9, 13.7, 14.9, 15.3, 20.2 or 21.6° ± 0.2° (2-theta). Precisely, the amorphous lorcaserin hydrochloride of the present invention is characterized by having an X-ray powder diffraction (XRPD) pattern showing no peaks with a signal-to-noise (S/N) ratio equal or higher than 3.0 at 11.4, 11.5, 12.9, 13.7, 14.9, 15.3, 20.2 or 21.6° ± 0.2° (2-theta).

In a preferred embodiment, the pharmaceutical excipient is selected from the group comprising polyvinylpyrrolidone, polyethylene glycol, poly(vinyl caprolactam), polyvinyl acetate, polyethylene oxide, a synthetic cellulose derivative, an aminoalkyl methacrylate copolymer, silica, and mixtures thereof.

In a further preferred embodiment, the synthetic cellulose derivative is selected from the group comprising ethyl cellulose, cellulose acetate, cellulose acetate butyrate, cellulose acetate phthalate, hydroxypropyl methyl cellulose, hydroxypropyl methyl cellulose acetate phthalate, hydroxypropyl methyl cellulose acetate succinate, sodium carboxymethyl cellulose, and mixtures thereof, and preferably comprises hydroxypropyl methyl cellulose.

In a preferred embodiment, the mixture as hereinbefore described is characterized by having a concentration of amorphous lorcaserin hydrochloride in the range from 0.1 % to 60% (w/w), preferably from 1% to 50% (w/w), more preferably from 2% to 40% (w/w), and even more preferably from 5% to 30% (w/w).

The amorphous phase can be thought of as a frozen or supercooled liquid, but with the thermal fluctuations present in a liquid frozen to a greater or lesser extent, leaving only largely static structural disorder. One consequence of a disordered structure is that amorphous phases are thermodynamically unstable and, therefore, they are the most energetic forms of a compound. The tendency of amorphous phases is thus to revert to a more stable, crystalline form. The inherent physical and chemical instability associated with amorphous phases renders them less desirable. The main issues surrounding instability arise from spontaneous crystallization upon storage of the drug, crystallization or chemical instability in formulations, and secondary processing (see M. Gibson, "Pharmaceutical Preformulation and Formulation", 2^{nd} edition, Informa Healthcare, page 62).

In this sense, the author of the present invention has surprisingly found that the amorphous lorcaserin hydrochloride of the present invention shows an improved physical stability when compared with the crystalline forms of lorcaserin hydrochloride. Particularly, the amorphous lorcaserin hydrochloride of the present invention does not show any polymorphic transformation *(i.e.* crystallization) or chemical degradation when heated up to a temperature of 50 °C, even under vacuum and in the presence of phosphorus pentoxide, while crystalline Form III (hemihydrate) as disclosed in U.S. Patent No. 8,168,624 and patent application WO 2011/153206 A1 is known to partially convert into anhydrous Form I after heating. Drying steps are usually included in the final manufacturing steps of both active pharmaceutical ingredients and final dosage forms, in order to remove residual solvents from the manufacturing process. Polymorphic forms typically differ in their physical properties such as, for example, melting point, solubility, and chemical reactivity. Particularly, the characteristics of the respective polymorphic forms can appreciably influence the solubility profile of a chemical substance. Further, the particular characteristics of the respective polymorphic forms can appreciably influence pharmaceutical properties such as dissolution rate and bioavailability. Consequently, polymorphic transformations during the manufacturing process are not desired.

On the other hand, the amorphous lorcaserin hydrochloride of the present invention does not show any polymorphic transformation *(i.e.* crystallization) or chemical degradation when stored under air atmosphere at 40 °C and 75% relative humidity, while the anhydrous crystalline forms of lorcaserin hydrochloride *(i.e.* anhydrous Form I as disclosed in U.S. Patent No. 8,168,624, which is equivalent to anhydrous Form II as disclosed in WO 2011/153206 A1; anhydrous Form II as disclosed in U.S. Patent No. 8,168,624; anhydrous Form I as disclosed in WO 2011/153206 A1; and anhydrous Form IV as disclosed in WO 2011/153206 A1) are known to readily convert to the hemihydrate (Form III) form upon exposure to air moisture. Contact with air moisture is likely to happen during the manipulation and storage of active pharmaceutical ingredients and final dosage forms under normal conditions. Since the particular characteristics of the respective polymorphic forms can appreciably influence pharmaceutical properties such as dissolution rate and bioavailability, polymorphic transformations during the manufacturing process or the storage are not desired.

Despite its inherent stability issues, amorphous phases are usually investigated if the crystalline forms of the active pharmaceutical ingredient are low soluble in aqueous media, since the predicted solubility of amorphous phases is usually between 4 to 25 times higher than the solubility of the crystalline phases. However, crystalline lorcaserin hydrochloride hemihydrate is a highly soluble and highly permeable (BCS Class 1) compound, with solubility in water greater than 400 mg/mL, and consequently the skilled person would not be motivated to obtain amorphous lorcaserin hydrochloride.

Similarly, the author of the present invention has surprisingly found that the mixture comprising amorphous lorcaserin hydrochloride of the present invention does not show any polymorphic transformation *(i.e.* crystallization) or chemical degradation when heated up to a temperature of 50 °C, even under vacuum and in the presence of phosphorus pentoxide, and that the mixture comprising amorphous lorcaserin hydrochloride of the present invention does not show any polymorphic transformation *(i.e.* crystallization) or chemical degradation when stored under air atmosphere at 40 °C and 75% relative humidity.

Similarly, the author of the present invention has surprisingly found that a pharmaceutical composition comprising amorphous lorcaserin hydrochloride of the present invention does not show any polymorphic transformation *(i.e.* crystallization) or chemical degradation when heated up to a temperature of 50 °C, even under vacuum and in the presence of phosphorus pentoxide, and that the mixture comprising amorphous lorcaserin hydrochloride of the present invention does not show any polymorphic transformation *(i.e.* crystallization) or chemical degradation when stored under air atmosphere at 40 °C and 75% relative humidity.

The present invention further provides a process for the preparation of the mixture comprising amorphous lorcaserin hydrochloride as hereinbefore described, the process comprising:
(a) contacting lorcaserin hydrochloride with at least one pharmaceutical excipient, in the presence of a suitable solvent; and
(b) removing the solvent.

In a preferred embodiment, the pharmaceutical excipient is selected from the group comprising polyvinylpyrrolidone, polyethylene glycol, poly(vinyl caprolactam), polyvinyl acetate, polyethylene oxide, a synthetic cellulose derivative, an aminoalkyl methacrylate copolymer, silica, and mixtures thereof.

In a further preferred embodiment, the synthetic cellulose derivative is selected from the group comprising ethyl cellulose, cellulose acetate, cellulose acetate butyrate, cellulose acetate phthalate, hydroxypropyl methyl cellulose, hydroxypropyl methyl cellulose acetate phthalate, hydroxypropyl methyl cellulose acetate succinate, sodium carboxymethyl cellulose, and mixtures thereof, and preferably comprises hydroxypropyl methyl cellulose.

In a preferred embodiment, the solvent is selected from the group comprising acetic acid, acetone, 1-butanol, 2-butanol, ethanol, formic acid, 3-methyl-1-butanol, 2-methyl-1-propanol, 1-pentanol, 1-propanol, 2-propanol, water, and mixtures thereof, and preferably comprises water.

In a preferred embodiment, the step of removing the solvent comprises evaporation, spray drying or lyophilization, and preferably comprises spray drying.

The present invention further provides a process for the preparation of the mixture comprising amorphous lorcaserin hydrochloride as hereinbefore described, the process comprising:
(a) contacting lorcaserin hydrochloride with at least one pharmaceutical excipient; and
(b) mechanically comminuting the resulting mixture.

In a preferred embodiment, the pharmaceutical excipient is selected from the group comprising polyvinylpyrrolidone, polyethylene glycol, poly(vinyl caprolactam), polyvinyl acetate, polyethylene oxide, a synthetic cellulose derivative, an aminoalkyl methacrylate copolymer, silica, and mixtures thereof, and preferably comprises silica.

In a further preferred embodiment, the synthetic cellulose derivative is selected from the group comprising ethyl cellulose, cellulose acetate, cellulose acetate butyrate, cellulose acetate phthalate, hydroxypropyl methyl cellulose, hydroxypropyl methyl cellulose acetate phthalate, hydroxypropyl methyl cellulose acetate succinate, sodium carboxymethyl cellulose, and mixtures thereof.

In a preferred embodiment, the step of mechanically comminuting comprises milling or extruding, and preferably comprises milling.

The present invention further provides a pharmaceutical composition comprising the mixture of lorcaserin hydrochloride in amorphous form with at least one pharmaceutical excipient and optionally, one or more pharmaceutical excipients.

The present invention further provides the use of the amorphous lorcaserin hydrochloride as hereinbefore described in the manufacture of a medicament for chronic weight management. Particularly, the present invention provides the use of the amorphous lorcaserin hydrochloride as hereinbefore described in the manufacture of a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (e.g., hypertension, dyslipidemia, type 2 diabetes).

The present invention further provides the use of the mixture comprising amorphous lorcaserin hydrochloride and at least one pharmaceutical excipient as hereinbefore described in the manufacture of a medicament for chronic weight management. Particularly, the present invention provides the use of the mixture comprising amorphous lorcaserin hydrochloride as hereinbefore described in the manufacture of a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (e.g., hypertension, dyslipidemia, type 2 diabetes).

The present invention further provides the use of the pharmaceutical composition comprising amorphous lorcaserin hydrochloride as hereinbefore described in the manufacture of a medicament for chronic weight management. Particularly, the present invention provides the use of the pharmaceutical composition comprising amorphous lorcaserin hydrochloride as hereinbefore described in the manufacture of a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (e.g., hypertension, dyslipidemia, type 2 diabetes).

The term "about" when used in the present invention preceding a number and referred to it, is meant to designate any value which lies within the range defined by the number ±10% of its value, preferably a range defined by the number ±5%, more preferably a range defined by the number ±2%, still more preferably a range defined by the number ±1%. For example, "about 10" should be construed as meaning within the range of 9 to 11, preferably within the range of 9.5 to 10.5, more preferably within the range of 9.8 to 10.2, and still more preferably within the range of 9.9 to 10.1.

There is also provided by the present invention a cocrystal comprising lorcaserin and urea with improved properties, in particular regarding its handling and for the formulation of pharmaceutical compositions.

In another aspect, the present invention provides a cocrystal comprising lorcaserin and urea.

A cocrystal is a crystalline entity in which more than one molecular substance is incorporated into the unit cell, and where at least two of these molecular substances are solid components (*i.e.,* solid compounds in their pure forms at room temperature and pressure), and are held together by a weak interaction. Weak interaction is defined as an interaction which is neither ionic nor covalent and includes, for example, hydrogen bonds, van der Waals interactions, and π-π interactions. In the particular case of cocrystals comprising a pharmaceutical active ingredient, the solid components that are held to the active ingredient by a weak interaction are also known as coformers. Therefore, the definition of cocrystal excludes traditional solvates, which are associations of substrates with solvents from which they are crystallized, since in this case the substrate is held by a weak interaction only to a molecule which is not a solid compound in their pure form at ambient conditions. However, in addition to the at least two solid components, cocrystals may also include solvent molecules in the crystalline lattice. Similarly, the definition of cocrystal also excludes traditional salts, which are distinguished by having all their solid components held by proton transfer, giving electrostatic linkage between oppositely-charged ions. However, a cocrystal may also have some of their components held by proton transfer, provided that at least another solid component is held by a weak interaction.

Urea (diaminomethanal) is a solid compound at room temperature (melting point 133-135 °C). It is a practically non-ionizable compound, highly soluble in water and practically non-toxic since it plays an important role in the metabolism of nitrogen-containing compounds by animals.

In a preferred embodiment, the cocrystal comprising lorcaserin and urea of the present invention is characterized by having a molar ratio of lorcaserin to urea comprised between 0.8 and 1.2, preferably between 0.9 and 1.1. In a further preferred embodiment, the cocrystal comprising lorcaserin and urea of the present invention is characterized by having a molar ratio of lorcaserin to urea of 1.0.

As used in the present invention, the molar ratio of lorcaserin to urea is calculated as the number of molecules of lorcaserin within the unit cell of the cocrystal, divided by the number of molecules of urea within the same unit cell of the cocrystal. It can be estimated by quantifying the amount of lorcaserin and the amount of urea that is contained in a certain amount of cocrystal, for example by using a suitable HPLC quantification method.

In a preferred embodiment, the cocrystal comprising lorcaserin and urea of the present invention further comprises hydrogen chloride. In a further preferred embodiment, the cocrystal of the present invention comprises lorcaserin in form of lorcaserin hydrochloride, so that the secondary amine moiety of lorcaserin is in protonated form and there is also a chloride anion within the crystalline structure of the cocrystal.

In a preferred embodiment, the cocrystal comprising lorcaserin and urea of the present invention further comprises a solvent, which is preferably selected from the group comprising acetone, acetonitrile, anisole, 1-butanol, 2-butanol, butyl acetate, *tert-*butyl methyl ether, cumene, cyclopentyl methyl ether, 1,4-dioxane, ethanol, ethyl acetate, ethyleneglycol, ethyl ether, ethyl formate, heptane, isobutyl acetate, isopropanol, isopropyl acetate, isopropyl ether, methanol, methyl acetate, 3-methyl-1-butanol, methylcyclohexane, methyl ethyl ketone, methyl isobutyl ketone, methyl isopropyl ketone, 2-methyl-1-propanol, 2-methyltetrahydrofuran, 1-pentanol, 1-propanol, propyl acetate, tetrahydrofuran, toluene, water, xylene, and/or mixtures thereof. In a further preferred embodiment, the molar ratio of solvent to lorcaserin within the unit cell is comprised between 0.1 and 10. As used in the present invention, the molar ratio of solvent to lorcaserin is calculated as the number of molecules of solvent within the unit cell of the cocrystal, divided by the number of molecules of lorcaserin within the same unit cell of the cocrystal.

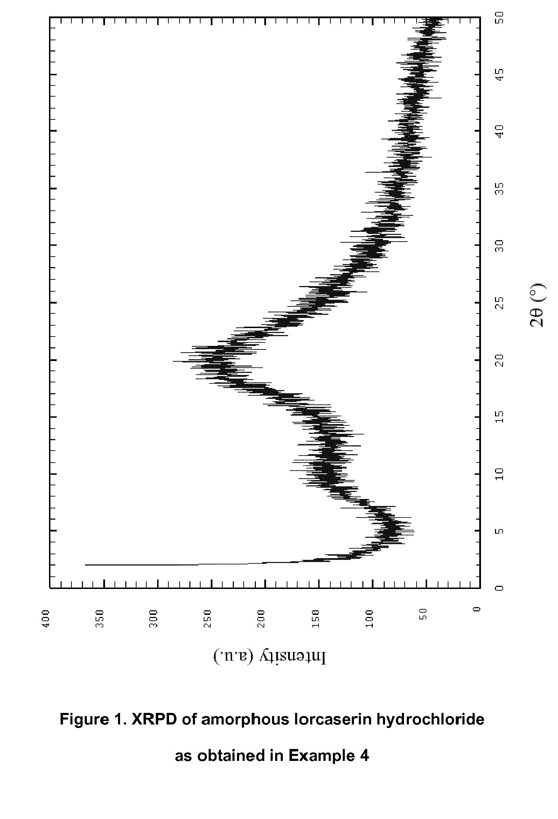

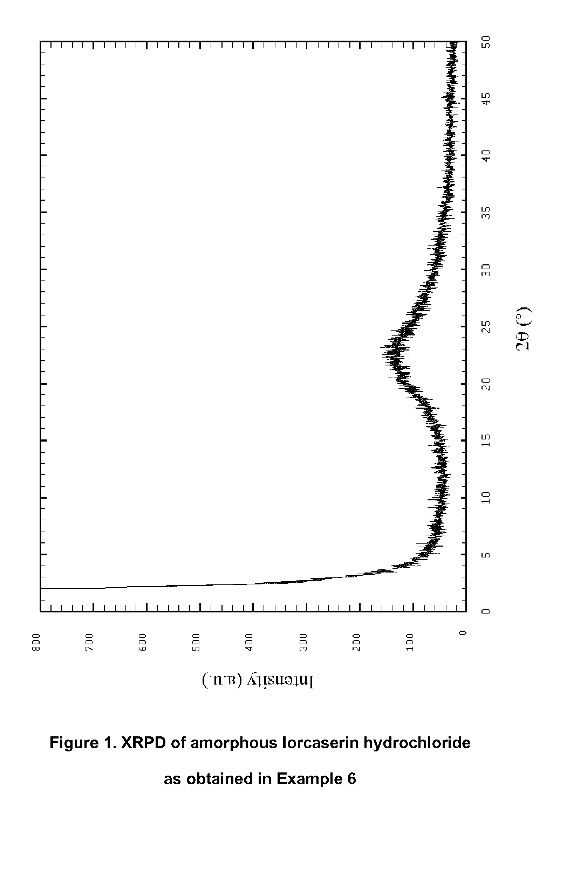

In a preferred embodiment, the cocrystal comprising lorcaserin and urea of the present invention is characterized by having an X-ray powder diffraction (XRPD) pattern comprising peaks at 13.4, 16.3 and 23.3° ± 0.2° 2-theta (2θ), preferably comprising peaks at 19.7, 21.5, 24.4, 24.7 and 33.0° ± 0.2° 2-theta (2θ), and more preferably comprising peaks at 8.2, 11.4, 13.4, 15.6, 16.3, 17.2, 19.7, 21.5, 21.9, 22.4, 23.1, 23.3, 24.0, 24.4, 24.7, 25.5, 25.7, 27.1, 28.3, 29.2, 30.0, 30.8, 31.1 and 33.0° ± 0.2° 2-theta (2θ). In a further preferred embodiment, the cocrystal comprising lorcaserin and urea of the present invention is characterized by having an X-ray powder diffraction (XRPD) pattern substantially equivalent to the diffraction plot depicted in Figure 1.

The present invention further provides a process for the preparation of the cocrystal comprising lorcaserin and urea as hereinbefore described, the process comprising contacting lorcaserin and urea, optionally in the presence of the suitable solvent.

In a preferred embodiment, the process as hereinbefore described comprises the use of lorcaserin in a molar ratio between 0.8 and 1.2 with respect to urea, preferably between 0.9 and 1.1 with respect to urea. In a further preferred embodiment, the process as hereinbefore described comprises the use of lorcaserin in a molar ratio of 1.0 with respect to urea.

In a preferred embodiment, the process as hereinbefore described further comprises the use of hydrogen chloride. In a further preferred embodiment, lorcaserin is used in form of lorcaserin hydrochloride in the process as hereinbefore described.

In a preferred embodiment, lorcaserin hydrochloride is used in anhydrous form in the process as hereinbefore described. In a further preferred embodiment, anhydrous lorcaserin hydrochloride that is used in the process as hereinbefore described is selected from the group comprising Form I as disclosed in U.S. Patent No. 8,168,624, Form II as disclosed in U.S. Patent No. 8,168,624, Form I as disclosed in patent application WO 2011/153206 A1, Form II as disclosed in patent application WO 2011/153206 A1, Form IV as disclosed in patent application WO 2011/153206 A1, and/or mixtures thereof.

In a preferred embodiment, the solvent is selected from the group comprising acetone, acetonitrile, anisole, 1-butanol, 2-butanol, butyl acetate, *tert-*butyl methyl ether, cumene, cyclopentyl methyl ether, 1,4-dioxane, ethanol, ethyl acetate, ethyleneglycol, ethyl ether, ethyl formate, heptane, isobutyl acetate, isopropanol, isopropyl acetate, isopropyl ether, methanol, methyl acetate, 3-methyl-1-butanol, methylcyclohexane, methyl ethyl ketone, methyl isobutyl ketone, methyl isopropyl ketone, 2-methyl-1-propanol, 2-methyltetrahydrofuran, 1-pentanol, 1-propanol, propyl acetate, tetrahydrofuran, toluene, water, xylene, and/or mixtures thereof.

In a preferred embodiment, the process as hereinbefore described further comprises a step of removing the solvent. In a further preferred embodiment, the solvent is removed by filtration and/or evaporation, preferably by filtration.

In a preferred embodiment, the step of contacting lorcaserin and urea of the process as hereinbefore described comprises milling a solid mixture comprising lorcaserin and urea.

The present invention still further provides a pharmaceutical composition comprising the cocrystal comprising lorcaserin and urea as hereinbefore described, together with at least one pharmaceutically acceptable excipient.

The present invention further provides a cocrystal comprising lorcaserin and urea as hereinbefore described, for use in a medicament for chronic weight management. Particularly, the present invention provides a cocrystal comprising lorcaserin and urea as hereinbefore described, for use in a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g*., hypertension, dyslipidemia, type 2 diabetes).

The present invention still further provides a pharmaceutical composition comprising the cocrystal as hereinbefore described, for use in a medicament for chronic weight management. Particularly, the present invention provides a pharmaceutical composition comprising the cocrystal as hereinbefore described, for use in a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

The present invention further provides the use of the cocrystal comprising lorcaserin and urea as hereinbefore described for the treatment of chronic weight management. Particularly, the present invention provides the use of the cocrystal comprising lorcaserin and urea as hereinbefore described as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

The present invention still further provides the use of the pharmaceutical composition comprising the cocrystal as hereinbefore described for the treatment of chronic weight management. Particularly, the present invention provides the use of the pharmaceutical composition comprising the cocrystal as hereinbefore described as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

There is also provided by the present invention a cocrystal comprising lorcaserin and mandelic acid with improved properties, in particular regarding stability, solubility, handling and improved properties for the formulation of pharmaceutical compositions.

Mandelic acid is the common name for 2-hydroxy-2-phenylacetic acid. It is a chiral molecule which exists in either of two enantiomers as well as the racemic mixture. L-(+)-mandelic acid corresponds to (*S*)-2-hydroxy-2-phenylacetic acid, while D-(-)-mandelic acid corresponds to (*R*)-2-hydroxy-2-phenylacetic acid, and both are solid compounds with a melting point of 132-135 °C. Racemic mandelic acid is also a solid compound with a melting point of 119 °C. It is a nontoxic substance that, after being ingested orally, is excreted unchanged in the urine.

In a preferred embodiment, mandelic acid comprises L-(+)-mandelic acid. In a further preferred embodiment, mandelic acid is L-(+)-mandelic acid.

In a preferred embodiment, the cocrystal comprising lorcaserin and mandelic acid of the present invention is characterized by having a molar ratio of lorcaserin to mandelic acid comprised between 0.8 and 1.2, preferably between 0.9 and 1.1. In a further preferred embodiment, the cocrystal comprising lorcaserin and mandelic acid of the present invention is characterized by having a molar ratio of lorcaserin to mandelic acid of 1.0.

As used in the present invention, the molar ratio of lorcaserin to mandelic acid is calculated as the number of molecules of lorcaserin within the unit cell of the cocrystal, divided by the number of molecules of mandelic acid within the same unit cell of the cocrystal. It can be estimated by quantifying the amount of lorcaserin and the amount of mandelic acid that is contained in a certain amount of cocrystal, for example by using a suitable HPLC quantification method.

In a preferred embodiment, the cocrystal comprising lorcaserin and mandelic acid of the present invention further comprises hydrogen chloride. In a further preferred embodiment, the cocrystal of the present invention comprises lorcaserin in form of lorcaserin hydrochloride, so that the secondary amine moiety of lorcaserin is in protonated form and there is also a chloride anion within the crystalline structure of the cocrystal.

In a preferred embodiment, the cocrystal comprising lorcaserin and mandelic acid of the present invention can optionally comprise a solvent, which is preferably selected from the group comprising acetone, acetonitrile, anisole, 1-butanol, 2-butanol, butyl acetate, *tert-butyl* methyl ether, cumene, cyclopentyl methyl ether, 1,4-dioxane, ethanol, ethyl acetate, ethyleneglycol, ethyl ether, ethyl formate, heptane, isobutyl acetate, isopropanol, isopropyl acetate, isopropyl ether, methanol, methyl acetate, 3-methyl-1-butanol, methylcyclohexane, methyl ethyl ketone, methyl isobutyl ketone, methyl isopropyl ketone, 2-methyl-1-propanol, 2-methyltetrahydrofuran, 1-pentanol, 1-propanol, propyl acetate, tetrahydrofuran, toluene, water, xylene, and/or mixtures thereof. In a further preferred embodiment, the molar ratio of solvent to lorcaserin within the unit cell is comprised between 0 and 10. As used in the present invention, the molar ratio of solvent to lorcaserin is calculated as the number of molecules of solvent within the unit cell of the cocrystal, divided by the number of molecules of lorcaserin within the same unit cell of the cocrystal.

In a preferred embodiment, the cocrystal comprising lorcaserin and mandelic acid of the present invention is characterized by having an X-ray powder diffraction (XRPD) pattern comprising peaks at 9.9, 18.0 and 26.6° ± 0.2° 2-theta (2θ), preferably comprising peaks at 5.6, 9.9, 13.5, 17.6, 18.0, 19.9, 20.8, 22.7, 23.5, 24.5, 26.6, 28.5 and 28.9° ± 0.2° 2-theta (2θ), and more preferably comprising peaks at 5.6, 8.8, 9.9, 11.0, 13.5, 15.3, 16.7, 17.3, 17.6, 18.0, 19.1, 19.9, 20.8, 22.0, 22.7, 23.2, 23.5, 23.9, 24.5, 26.3, 26.6, 27.8, 28.5, 28.9, 29.7, 30.3, 30.8, 31.6 and 32.0° ± 0.2° 2-theta (2θ). In a further preferred embodiment, the cocrystal comprising lorcaserin and mandelic acid of the present invention is characterized by having an X-ray powder diffraction (XRPD) pattern substantially equivalent to the diffraction plot depicted in Figure 1.

The present invention further provides a process for the preparation of the cocrystal comprising lorcaserin and mandelic acid as hereinbefore described, the process comprising contacting lorcaserin and mandelic acid, optionally in the presence of the suitable solvent.

In a preferred embodiment, the process as hereinbefore described comprises the use of lorcaserin in a molar ratio between 0.8 and 1.2 with respect to mandelic acid, preferably between 0.9 and 1.1 with respect to mandelic acid. In a further preferred embodiment, the process as hereinbefore described comprises the use of lorcaserin in a molar ratio of 1.0 with respect to mandelic acid.

In a preferred embodiment, the process as hereinbefore described further comprises the use of hydrogen chloride. In a further preferred embodiment, lorcaserin is used in form of lorcaserin hydrochloride in the process as hereinbefore described.

In a preferred embodiment, lorcaserin hydrochloride is used in anhydrous form in the process as hereinbefore described. In a further preferred embodiment, anhydrous lorcaserin hydrochloride that is used in the process as hereinbefore described is selected from the group comprising Form I as disclosed in U.S. Patent No. 8,168,624, Form II as disclosed in U.S. Patent No. 8,168,624, Form I as disclosed in patent application WO 2011/153206 A1, Form II as disclosed in patent application WO 2011/153206 A1, Form IV as disclosed in patent application WO 2011/153206 A1, and/or mixtures thereof.

In a preferred embodiment, the solvent is selected from the group comprising acetone, acetonitrile, anisole, 1-butanol, 2-butanol, butyl acetate, *tert*-butyl methyl ether, cumene, cyclopentyl methyl ether, 1,4-dioxane, ethanol, ethyl acetate, ethyleneglycol, ethyl ether, ethyl formate, heptane, isobutyl acetate, isopropanol, isopropyl acetate, isopropyl ether, methanol, methyl acetate, 3-methyl-1-butanol, methylcyclohexane, methyl ethyl ketone, methyl isobutyl ketone, methyl isopropyl ketone, 2-methyl-1-propanol, 2-methyltetrahydrofuran, 1-pentanol, 1-propanol, propyl acetate, tetrahydrofuran, toluene, water, xylene, and/or mixtures thereof.

In a preferred embodiment, the process as hereinbefore described further comprises a step of removing the solvent. In a further preferred embodiment, the solvent is removed by filtration and/or evaporation, preferably by filtration.

In a preferred embodiment, the step of contacting lorcaserin and mandelic acid of the process as hereinbefore described comprises milling a solid mixture comprising lorcaserin and mandelic acid.

The present invention still further provides a pharmaceutical composition comprising the cocrystal comprising lorcaserin and mandelic acid as hereinbefore described, together with at least one pharmaceutically acceptable excipient.

The present invention further provides a cocrystal comprising lorcaserin and mandelic acid as hereinbefore described, for use in a medicament for chronic weight management. Particularly, the present invention provides a cocrystal comprising lorcaserin and mandelic acid as hereinbefore described, for use in a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

The present invention still further provides a pharmaceutical composition comprising the cocrystal as hereinbefore described, for use in a medicament for chronic weight management. Particularly, the present invention provides a pharmaceutical composition comprising the cocrystal as hereinbefore described, for use in a medicament to be used as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

The present invention further provides the use of the cocrystal comprising lorcaserin and mandelic acid as hereinbefore described for the treatment of chronic weight management. Particularly, the present invention provides the use of the cocrystal comprising lorcaserin and mandelic acid as hereinbefore described as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g*., hypertension, dyslipidemia, type 2 diabetes).

The present invention still further provides the use of the pharmaceutical composition comprising the cocrystal as hereinbefore described for the treatment of chronic weight management. Particularly, the present invention provides the use of the pharmaceutical composition comprising the cocrystal as hereinbefore described as an adjunct to a reduced-calorie diet and increased physical activity for chronic weight management in adult patients with an initial body mass index (BMI) of 30 kg/m² or greater (obese), or 27 kg/m² or greater (overweight) in the presence of at least one weight related comorbid condition (*e.g.*, hypertension, dyslipidemia, type 2 diabetes).

The following examples further illustrate the invention but, of course, should not be construed as in any way limiting its scope.

### EXAMPLES

### General Experimental Conditions:

The lorcaserin hydrochloride used as starting material in the examples below can be obtained by any of the synthetic processes disclosed in the literature. The HPLC purity of the lorcaserin hydrochloride that was used as a starting material in the examples below was higher than 99.5%.

### X-Ray Powder Diffraction (XRPD):

The XRPD pattern was recorded on a Siemens D5000 diffractometer equipped with two symmetrically mounted vertical goniometers (Bragg-Brentano geometry) with horizontal sample stages, a X-ray tube, a high voltage generator (working at 45 kV and 35 mA) and standard scintillation detectors. Ni-filtered Cu-anode source was used and diffracted radiation was further monochromatized with a graphite crystal to avoid fluorescence effects [(λ(K_{α}) = 1.54056 Å]. The diffraction pattern was recorded including values of 2θ that range from 2 to 50° with a sampling rate of 0.02° per second and a step time of 1 second per step. The powdered sample was pressed between two glass plates, forming a film. DIFFRAC Plus measurement software with EVA evaluation software (Bruker) was used to record the data and for a primary analysis of the diffraction pattern. The equipment was periodically calibrated using quartz and silicon.

### Example 1: Preparation of amorphous lorcaserin hydrochloride.

204 mg of anhydrous lorcaserin hydrochloride and 800 mg of polyvinylpyrrolidone (Plasdone^{™} K-29/32) were dissolved in 2 mL of deionized water at 20-25 °C. The solvent was evaporated at 50 °C under vacuum, in the presence of phosphorus pentoxide, and a solid was obtained. XRPD: Amorphous.

### Example 2: Preparation of amorphous lorcaserin hydrochloride.

201 mg of anhydrous lorcaserin hydrochloride and 802 mg of hydroxypropyl methyl cellulose (Pharmacoat^{™} 606) were dissolved in 4 mL of deionized water at 20-25 °C. The solvent was evaporated at 50 °C under vacuum, in the presence of phosphorus pentoxide, and a solid was obtained. XRPD: Amorphous.

### Example 3: Preparation of amorphous lorcaserin hydrochloride.

200 mg of anhydrous lorcaserin hydrochloride and 802 mg of an aminoalkyl methacrylate copolymer (Eudragit^{™} E PO) were dissolved in 2 mL of methanol at 20-25 °C. The solvent was evaporated under vacuum, and a solid was obtained. XRPD: Amorphous.

### Example 4: Preparation of amorphous lorcaserin hydrochloride.

801 mg of anhydrous lorcaserin hydrochloride were added to a previously prepared solution of 7.2 g of hydroxypropyl methyl cellulose (Pharmacoat^{™} 606) in 85 mL of deionized water at 20-25 °C. 15 mL of deionized water were added. The resulting solution was spray-dried using a Buchi Mini Spray Dryer B-290 operating under the following conditions: inlet temperature: 165 °C; outlet temperature: 100 °C; aspirator: 100 %; pump: 10 %; nozzle cleaner: 5. The obtained solid was further dried in a vacuum oven at 50 °C until constant weight, to obtain 6.0 g of solid. XRPD: Amorphous (see Figure 1).

### Example 5: Preparation of amorphous lorcaserin hydrochloride.

1.6 g of anhydrous lorcaserin hydrochloride were added to a previously prepared solution of 6.4 g of hydroxypropyl methyl cellulose (Pharmacoat^{™} 606) in 75 mL of deionized water at 20-25 °C. 25 mL of deionized water were added. The resulting solution was spray-dried using a Buchi Mini Spray Dryer B-290 operating under the following conditions: inlet temperature: 165 °C; outlet temperature: 89 °C; aspirator: 100 %; pump: 10 %; nozzle cleaner: 5. The obtained solid was further dried in a vacuum oven at 50 °C until constant weight, to obtain 6.0 g of solid. XRPD: Amorphous.

### Example 6: Preparation of amorphous lorcaserin hydrochloride.

336 mg of anhydrous lorcaserin hydrochloride (corresponding to Form I of WO 2011/153206 A1) were mixed with 3.0 g of silica gel (Syloid^{™} 244FP), and the resulting mixture was milled portionwise (12 parts) in a Retsch MM 400 ball mill operating at a frequency of 20 Hz (20 s⁻¹), in the presence of two 7 mm diameter agate beads, for 1 hour. After combining the milled products, 3.4 g of solid were obtained. XRPD: Amorphous (see Figure 2).

### Example 7: Preparation of amorphous lorcaserin hydrochloride.

750 mg of anhydrous lorcaserin hydrochloride (corresponding to Form I of WO 2011/153206 A1) were mixed with 3.0 g of silica gel (Syloid^{™} 244FP), and the resulting mixture was milled portionwise (12 parts) in a Retsch MM 400 ball mill operating at a frequency of 20 Hz (20 s⁻¹), in the presence of two 7 mm diameter agate beads, for 1 hour. After combining the milled products, 3.4 g of solid were obtained. XRPD: Amorphous.

### Example 8: Preparation of amorphous lorcaserin hydrochloride.

63 mg of anhydrous lorcaserin hydrochloride (corresponding to Form I of WO 2011/153206 A1) were mixed with 251 mg of silica gel (Syloid^{™} 244FP), and the resulting mixture was milled in a Retsch MM 400 ball mill operating at a frequency of 20 Hz (20 s⁻¹), in the presence of two 7 mm diameter agate beads, for 1 hour. A solid was obtained. XRPD: Amorphous.

### Example 9: Preparation of amorphous lorcaserin hydrochloride.

388 mg of anhydrous lorcaserin hydrochloride (corresponding to Form I of WO 2011/153206 A1) were mixed with 3.5 g of silica gel (Syloid^{™} XDP), and the resulting mixture was milled portionwise (7 parts) in a Retsch MM 400 ball mill operating at a frequency of 20 Hz (20 s⁻¹), in the presence of two 7 mm diameter agate beads, for 1 hour. After combining the milled products, 3.8 g of solid were obtained. XRPD: Amorphous.

### Example 10: Preparation of amorphous lorcaserin hydrochloride.

877 mg of anhydrous lorcaserin hydrochloride (corresponding to Form I of WO 2011/153206 A1) were mixed with 3.5 g of silica gel (Syloid^{™} 244FP), and the resulting mixture was milled portionwise (7 parts) in a Retsch MM 400 ball mill operating at a frequency of 20 Hz (20 s⁻¹), in the presence of two 7 mm diameter agate beads, for 1 hour. After combining the milled products, 4.3 g of solid were obtained. XRPD: Amorphous.

### Example 11: Preparation of amorphous lorcaserin hydrochloride.

126 mg of anhydrous lorcaserin hydrochloride (corresponding to Form I of WO 2011/153206 A1) and 501 mg of silica gel (Syloid^{™} XDP) was milled in a Retsch MM 400 ball mill operating at a frequency of 20 Hz (20 s⁻¹), in the presence of two 7 mm diameter agate beads, for 1 hour. A solid was obtained. XRPD: Amorphous.

### Examples 12 to 14: Stability study of amorphous lorcaserin hydrochloride.

Samples of amorphous lorcaserin hydrochloride as obtained in examples 2, 4 and 5 were exposed to air atmosphere at 40 °C and 75 % relative humidity, for 10 days. XRPD and HPLC analysis of the obtained samples did not show any substantial difference with respect to the initial samples (see Table 2).

**Table 2**

| Example | Initial sample | Result after 10 days at 40 °C, 75 % RH | |
|---|---|---|---|
| | | XRPD | Purity (HPLC) |
| 12 | Example 2 | Amorphous | 100% |
| 13 | Example 8 | Amorphous | 100% |
| 14 | Example 11 | Amorphous | 100% |

### Example 15: Preparation of the cocrystal of lorcaserin hydrochloride and urea.

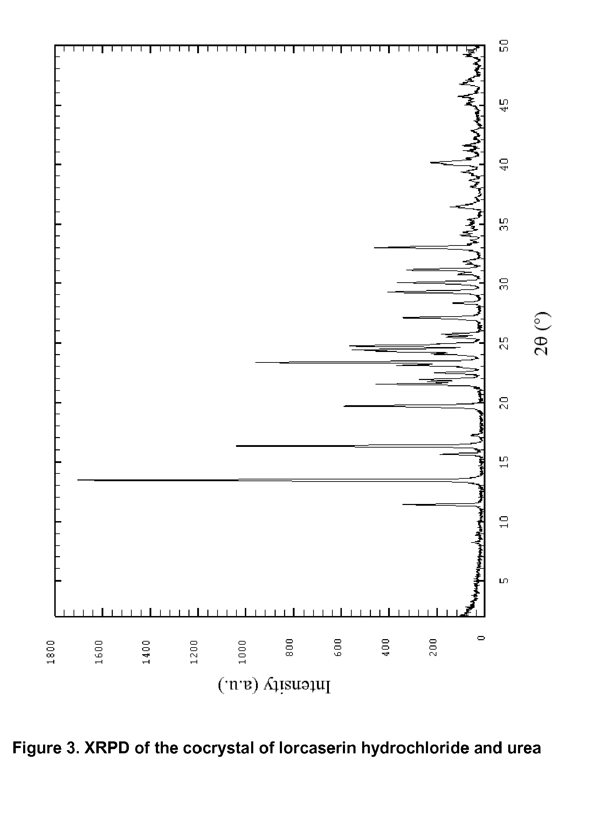

A mixture of 300.9 mg of anhydrous lorcaserin hydrochloride (corresponding to Form I of WO 2011/153206 A1), 79.7 mg of urea and two drops of acetone was milled in a Retsch MM 400 ball mill operating at a frequency of 20 Hz (20 s⁻¹), in the presence of two 7 mm diameter agate beads, for 30 minutes. A solid was obtained. XRPD: Substantially identical to Figure 3.

### Example 16: Preparation of the cocrystal of lorcaserin hydrochloride and urea.

A mixture of 301.6 mg of anhydrous lorcaserin hydrochloride (corresponding to Form I of WO 2011/153206 A1), 79.9 mg of urea and two drops of methanol was milled in a Retsch MM 400 ball mill operating at a frequency of 20 Hz (20 s⁻¹), in the presence of two 7 mm diameter agate beads, for 30 minutes. A solid was obtained. XRPD: Substantially identical to Figure 3.

### Example 17: Preparation of the cocrystal of lorcaserin hydrochloride and urea.

A mixture of 301.0 mg of anhydrous lorcaserin hydrochloride (corresponding to Form I of WO 2011/153206 A1) and 80.1 mg of urea was suspended in 2 mL of isopropanol and stirred at 20-25 °C for 24 hours. The resulting suspension was filtered and 290.4 mg of solid were obtained. XRPD: Substantially identical to Figure 3.

### Example 18: Preparation of the cocrystal of lorcaserin hydrochloride and urea.

A mixture of 301.4 mg of anhydrous lorcaserin hydrochloride (corresponding to Form I of WO 2011/153206 A1) and 80.3 mg of urea was suspended in 2 mL of ethanol and stirred at 20-25 °C for 1 hour. The resulting suspension was filtered and 175.6 mg of solid were obtained. XRPD: Substantially identical to Figure 3.

### Example 19: Preparation of the cocrystal of lorcaserin hydrochloride and urea.

A mixture of 301.0 mg of anhydrous lorcaserin hydrochloride (corresponding to Form I of WO 2011/153206 A1) and 79.7 mg of urea was dissolved in 6 mL of ethanol at 20-25 °C. 10 mL of methyl *tert-butyl* ether were added dropwise, and the resulting suspension was stirred at 20-25 °C for 1 hour. The suspension was filtered and 144.8 mg of solid were obtained. XRPD: Substantially identical to Figure 3.

### Example 20: Preparation of the cocrystal of lorcaserin hydrochloride and urea.

A mixture of 300.0 mg of anhydrous lorcaserin hydrochloride (corresponding to Form I of WO 2011/153206 A1) and 80.2 mg of urea was suspended in 1 mL of ethanol. The suspension was heated to 65 °C and a solution was obtained. 5 mL of methyl *tert-butyl* ether were added dropwise, and the resulting suspension was cooled to 20-25 °C and stirred at this temperature for 90 minutes. The suspension was filtered and 348.3 mg of solid were obtained. XRPD: See Figure 3.

### Example 21: Preparation of the cocrystal of lorcaserin hydrochloride and urea.

A mixture of 301.1 mg of anhydrous lorcaserin hydrochloride (corresponding to Form I of WO 2011/153206 A1) and 80.2 mg of urea was suspended in 2.5 mL of isopropanol. The suspension was heated to 70 °C and a solution was obtained. 5 mL of methyl *tert-butyl* ether were added dropwise, and the resulting suspension was cooled to 20-25 °C and stirred at this temperature for 1 hour. The suspension was filtered and 316.6 mg of solid were obtained. XRPD: Substantially identical to Figure 3.

### Example 22: Preparation of the cocrystal of lorcaserin hydrochloride and L-(+)-mandelic acid.

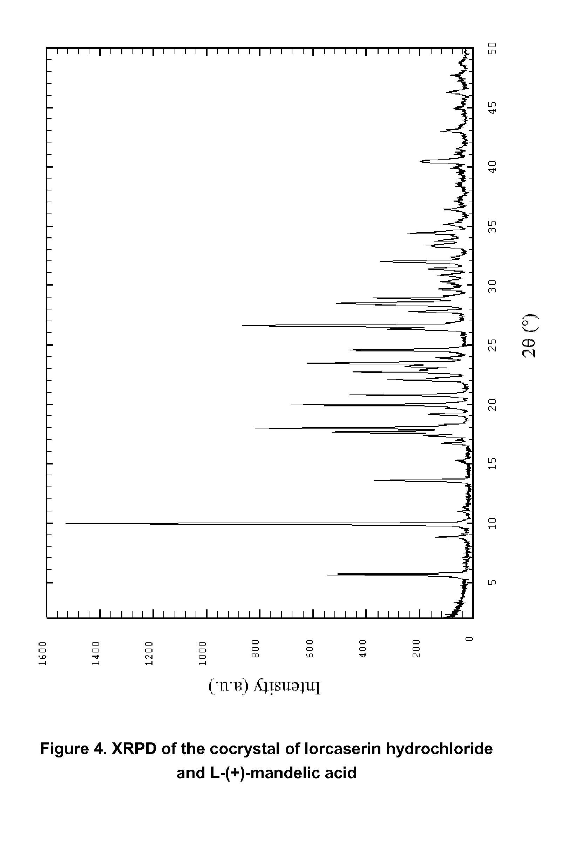

A mixture of 300.1 mg of anhydrous lorcaserin hydrochloride (corresponding to Form I of WO 2011/153206 A1), 196.3 mg of L-(+)-mandelic acid and two drops of ethyl acetate was milled in a Retsch MM 400 ball mill operating at a frequency of 20 Hz (20 s⁻¹), in the presence of two 7 mm diameter agate beads, for 30 minutes. A solid was obtained. XRPD: Substantially identical to Figure 4.

### Example 23: Preparation of the cocrystal of lorcaserin hydrochloride and L-(+)-mandelic acid.

A mixture of 301.3 mg of anhydrous lorcaserin hydrochloride (corresponding to Form I of WO 2011/153206 A1) and 198.0 mg of L-(+)-mandelic acid was dissolved in 1.2 mL of isopropanol at 20-25 °C. 4 mL of methylcyclohexane were added, and the resulting solution was seeded with cocrystal of lorcaserin hydrochloride and L-(+)-mandelic acid. The resulting suspension was stirred at 20-25 °C for 3 hours and filtered to obtain 150.5 mg of solid. XRPD: Figure 4.

## Claims

1. Amorphous lorcaserin hydrochloride.

2. The amorphous lorcaserin hydrochloride according to claim 1, **characterized by** having an X-ray powder diffraction pattern showing no detectable peaks in the range from 2 to 50° (2-theta).

3. A mixture comprising the amorphous lorcaserin hydrochloride of any of claims 1 to 2, together with at least one pharmaceutical excipient.

4. The mixture according to claim 3, wherein the pharmaceutical excipient is selected from the group comprising polyvinylpyrrolidone, polyethylene glycol, poly(vinyl caprolactam), polyvinyl acetate, polyethylene oxide, a synthetic cellulose derivative, an aminoalkyl methacrylate copolymer, silica, and mixtures thereof.

5. The mixture according to any of claims 3 to 4, wherein the concentration of amorphous lorcaserin hydrochloride is in the range from 0.1% to 60% (w/w), preferably in the range from 5% to 30% (w/w).

6. A process for the preparation of the mixture according to any of claims 3 to 5, the process comprising:
(a) contacting lorcaserin hydrochloride with at least one pharmaceutical excipient, in the presence of a suitable solvent; and
(b) removing the solvent.

7. The process according to claim 6, wherein the pharmaceutical excipient is selected from the group comprising polyvinylpyrrolidone, polyethylene glycol, poly(vinyl caprolactam), polyvinyl acetate, polyethylene oxide, a synthetic cellulose derivative, an aminoalkyl methacrylate copolymer, silica, and mixtures thereof.

8. The process according to any of claims 6 to 7, wherein the solvent is selected from the group comprising acetic acid, acetone, 1-butanol, 2-butanol, ethanol, formic acid, 3-methyl-1-butanol, 2-methyl-1-propanol, 1-pentanol, 1-propanol, 2-propanol, water, and mixtures thereof.

9. A pharmaceutical composition comprising a mixture according to any of claims 3 to 8, optionally with one or more pharmaceutical excipients.

10. Amorphous lorcaserin hydrochloride according to claims 1 to 2 for its use in the treatment of chronic weight management.

11. A pharmaceutical composition according to claim 9 for its use in the treatment of chronic weight management.

12. A cocrystal comprising lorcaserin and urea.

13. The cocrystal according to claim 12, **characterized by** having an X-ray powder diffraction pattern comprising peaks at 13.4, 16.3 and 23.3° ± 0.2° 2-theta.

14. A cocrystal comprising lorcaserin and mandelic acid.

15. The cocrystal according to claim 14, **characterized by** having an X-ray powder diffraction pattern comprising peaks at 9.9, 18.0 and 26.6° ± 0.2° 2-theta.
